# EUROPEAN PATENT APPLICATION

(11) **EP 3 791 925 A1**
(43) Date of publication of application: **17.03.2021**
(21) Application number: 19199818.6
(22) Date of filing: 26.09.2019
(51) Int. Cl.: A61N 1/375, A61N 1/372, A61N 1/05

(54) **LEADLESS PACEMAKER AND METHODS FOR ELECTRICALLY STIMULATING CARDIAC TISSUE, SENSING ELECTRICAL SIGNALS AND COMMUNICATING BETWEEN A LEADLESS PACEMAKER AND AN EXTERNAL DEVICE**

(30) Priority: 11.09.2019 US 201962898563 P
(71) Applicant: BIOTRONIK SE & Co. KG, 12359 Berlin (DE)
(72) Inventor: Muessig, Dirk, West Linn, OR Oregon 97068 (US); Stotts, Larry, Tigard, OR Oregon 97224 (US)
(74) Representative: Galander, Marcus

(57) **Abstract**

The disclosure relates to a leadless pacemaker (1) comprising at least one fixation element (2) for fixating the leadless pacemaker to cardiac tissue, a communication unit (3) which is electrically connected to said fixation element (2), so that said fixation element (2) is configured to act as a communication antenna for transmitting signals generated by said communication unit (3) to an external device and/or receiving signals from an external device, and a therapy unit (4) for generating electrical signals to electrically stimulate cardiac tissue, wherein said fixation element (2) is configured to act as an electrode for electrically stimulating cardiac tissue and/or sensing electrical signals of the cardiac tissue. Furthermore, the disclosure relates to a method for electrically stimulating cardiac tissue, a method for sensing electrical signals of cardiac tissue by means of the leadless pacemaker (1), and a method for communicating between a leadless pacemaker (1) and an external device.

## Description

The disclosure relates to a leadless pacemaker, a method for electrically stimulating cardiac tissue, a method for sensing electrical signals of cardiac tissue and a method for communicating between a leadless pacemaker and an external device.

Pacemakers are implantable devices which deliver electrical pulses to the human or animal heart to stimulate the heart and maintain cardiac rhythm in patients with heart disease.

In contrast to traditional pacemakers which are implanted in a subcutaneous location, leadless pacemakers are small enough to be directly implanted into the heart, and therefore lack electrical leads guided from the pacemaker to the heart. This was made possible as technology advancements allowed to miniaturize the electronics and battery to a size that allows a direct implant into the ventricle of the patient.

Such leadless pacemakers are capable to provide an equivalent therapy compared to traditional pacemakers implanted in a subcutaneous location of the patient. However, due to the very limited volume of the leadless pacemaker and an implant location deep in the human body there are a number of major challenges related to such a device. One challenge is the way to communicate with the device, which has long been limited to nearfield coil communication.

Traditional pacemakers mostly offer wireless communication capabilities to external devices. The antenna required for communication is often located as a separate element in the header of the traditional pacemaker. Typical communication frequencies are within the MICS band (405MHz, MICS - Medical Implant Communication Service) or the Bluetooth frequency of 2.4 GHz.

Patent application US 2019/0030346 A1 describes a leadless pacemaker which uses a fixation element as an antenna to communicate wirelessly between a transceiver circuit of the pacemaker and an external device.

However, such a pacemaker is still relatively bulky and complex, necessitating further improvements to reduce size and complexity.

An objective can be seen in providing improved technologies for a leadless pacemaker, particularly a leadless pacemaker having a reduced size and complexity compared to the prior art.

This objective is obtained by the subject matter of the independent claims 1 (leadless pacemaker), 12 (method for electrically stimulating cardiac tissue), 13 (method for sensing electrical signals) and 15 (method for communicating between a leadless pacemaker and an external device). Further embodiments are subject matter of dependent claims.

A first aspect relates to a leadless pacemaker comprising at least one fixation element for fixating the leadless pacemaker to cardiac tissue, a communication unit which is electrically connected to the fixation element, so that the fixation element is configured to act as a communication antenna for transmitting signals generated by the communication unit to an external device and/or so that the fixation element is configured to act as a communication antenna for receiving signals from an external device, and a therapy unit for generating electrical signals to electrically stimulate cardiac tissue, wherein the fixation element is configured to act as an electrode for electrically stimulating cardiac tissue and/or the fixation element is configured to act as an electrode for sensing electrical signals of the cardiac tissue.

The term "fixating" herein designates tightly anchoring the leadless pacemaker at its intended position in the cardiac tissue, particularly in the myocardium, such that the pacemaker is not displaced during its service life. Fixation elements may be any suitable means of fixating the pacemaker in the cardiac tissue. They may consist of or comprise any suitable material and comprise any suitable shape. Examples of fixation elements are screws, hooks (also termed tines), barbs and sutures. The leadless pacemaker may comprise a single fixation element (e.g. a single screw or helix-type fixation element) or more than one fixation element (a plurality of fixation elements, e.g. a plurality of tines).

In particular, the leadless pacemaker may be installed in an inner wall of an atrium or an inner wall of a ventricle of the heart.

Furthermore, particularly, the leadless pacemaker extends along a longitudinal axis from a distal end to a proximal end. Therein, the distal end is the end of the pacemaker which is anchored in the cardiac tissue and the proximal end is the opposite end, which is proximal to the physician during implantation of the pacemaker. The fixation element (or plurality of fixation elements) is particularly positioned at the distal end.

The leadless pacemaker comprises a communication unit, in other words, a device capable of mediating wireless communication between the leadless pacemaker and other devices, e.g. an external device. A possible type of communication unit is a transceiver. For example, communication frequencies used for communication between the communication unit and the external device are within the MICS band (405MHz) or the Bluetooth frequency of 2.4 GHz.

In the context of the present specification, the term "external device" designates a device which is separate from (external of) the leadless pacemaker. Therefore, external devices may be separate devices implanted into the same human or animal body carrying the pacemaker, or devices positioned outside of this human or animal body. Example of external devices are diagnostic devices used during setup or maintenance of the leadless pacemaker.

The fixation element (or the plurality of fixation elements) serves as a communication antenna. In other words, the fixation element is configured to transmit electromagnetic waves used for wireless communication with the external device based on the signals generated by the communication unit and/or receive electromagnetic waves from the external device which are subsequently received by the communication unit to be further used or processed. To this end, the fixation element particularly consists of or comprises an electrically conductive material such as a metal, particularly a super-elastic material such as a form-shape alloy (e.g. Nitinol), and is electrically connected to the communication unit.

Further, the leadless pacemaker comprises a therapy unit. This unit may be any device capable of generating electrical signals for electrical stimulation of the heart by means of the fixation element serving as an electrode (alone or in combination with other electrodes). For example, the therapy unit may be a pulse generator. In addition, the therapy unit may also be capable of sensing electrical signals of the cardiac tissue using the fixation element as a sensing electrode.

The fixation element (or the plurality of fixation elements) fulfils a triple function: it anchors the leadless pacemaker in the cardiac tissue, it serves as a communication antenna for wireless communication, and it serves as an electrode for electrical stimulation of the heart and/or electrical sensing. By implementing these three functions in a single part, the size and complexity of the leadless pacemaker is significantly reduced compared to devices of the prior art.

In certain embodiments, the fixation element is electrically connected to the therapy unit, such that a voltage may be applied to the fixation element, particularly by the therapy unit. Therein, in particular, the fixation element is shaped as a helical screw or comprises a helical screw. More particularly, the fixation element extends from the distal end of the leadless pacemaker towards the distal direction and is helically wound around an imaginary axis, which is parallel to the longitudinal axis of the leadless pacemaker.

In other words, the fixation element (or the plurality of fixation elements) is used or usable as a pacing electrode to deliver an electric pulse to the cardiac tissue. The voltage applied to the fixation element is an electrical potential difference between the fixation element and ground potential, wherein the ground potential is particularly the electric potential of a housing of the leadless pacemaker or a separate counter-electrode, such as a ring electrode.

In certain embodiments, the leadless pacemaker comprises a signal splitter for separating signals of the communication unit from signals of the therapy unit, wherein the fixation element is electrically connected to the communication unit and the therapy unit via the signal splitter. Therein, in particular, the signals of the therapy unit and the communication unit are characterized by separate frequencies.

A signal splitter, in the context of the present specification, is any device capable of separating signals of the communication unit from signals of the therapy unit.

In certain embodiments, the leadless pacemaker comprises a pacing electrode, which is electrically connected to the therapy unit, wherein the fixation element is configured to act as a counter electrode, such that a voltage may be applied between the pacing electrode and the fixation element, particularly by the therapy unit. Therein, in particular, the fixation element comprises at least one hook for fixating the leadless pacemaker to the cardiac tissue, wherein the at least one hook is configured to act as a counter electrode, such that a voltage may be applied between the pacing electrode and the at least one hook.

According to this embodiment, the pacemaker comprises a fixation element and an additional pacing electrode separate from the fixation element. The fixation element (or the plurality of fixation elements) serves as a counter-electrode defining a ground potential, wherein a positive or negative electric potential is applied to a separate pacing electrode, such that a potential difference (voltage) is established between the pacing electrode and the fixation element to deliver an electric pulse to the cardiac tissue into which the leadless pacemaker is implanted.

Using the fixation element as a counter electrode eliminates the need for a separate designated counter-electrode, such as a proximal ring electrode, and therefore reduces size and complexity of the leadless pacemaker. In addition, the local resolution of the generated electric field is significantly improved compared to a field generated by a single pacing electrode with the housing of the pacemaker defining ground potential and compared to a field generated by a pacing electrode and a proximal ring electrode.

The pacing electrode is particularly a tip electrode. Therein, the term "tip electrode" particularly designates an electrode comprising a protrusion having a convex shape increasing the electric field at the protrusion when a positive or negative potential is applied to the electrode.

In certain embodiments, the leadless pacemaker further comprises a ring electrode and an electronic circuit, wherein the electronic circuit is configured to determine a far field signal of the cardiac tissue from a first electrical signal sensed between the pacing electrode and the fixation element, and a second electrical signal sensed between the pacing electrode and the ring electrode, particularly wherein the pacing electrode is positioned at a distal end of the leadless pacemaker and the ring electrode is positioned at a proximal end of the leadless pacemaker.

In the context of the present specification, the term "ring electrode" particularly designates an electrode extending in a circumferential direction relative to the longitudinal axis along which the leadless pacemaker extends.

The term "electrical signal sensed between" two electrodes in the context of the present specification means that an external electric field generated by the cardiac tissue is detected as a potential difference between the respective pair of electrodes.

In particular, in case the leadless pacemaker is implanted in a ventricle of the heart, the far field signal of the cardiac tissue may be the result of an electric signal generated in an atrium of the heart.

With conventional electrode setups according to the prior art, it is difficult to separate near field (e.g. ventricular) electric signals from far field (e.g. atrial) signals. However, in the described embodiment, a first electrode pair constituted by the pacing electrode and the fixation element is more sensitive to local electric fields generated close to the site of implantation, for instance the ventricle, compared to a second electrode pair constituted by the pacing electrode and the ring electrode. Therefore, it is possible to separate near field and far field signals by comparing and analyzing the difference between the signals of the two electrode pairs. This task is performed by the electronic circuit which is configured to analyze the near field signals and the far field signals.

In certain embodiments, the fixation element is shaped as a helical screw or comprises a helical screw. In particular, the fixation element extends from the distal end of the leadless pacemaker towards the distal direction and is helically wound around an imaginary axis, the axis being parallel to the longitudinal axis of the leadless pacemaker.

Advantageously, such a fixation element has a single tip extending in the distal direction and therefore is suitable to be used as a pacing electrode.

In certain embodiments, the fixation element comprises at least one hook for fixating the leadless pacemaker to said cardiac tissue, particularly wherein the at least one hook is positioned at the distal end of the leadless pacemaker.

In the context of the present specification, the term "hook" particularly describes a curved elongated object having an arc length and a width perpendicular to the arc length, wherein the ratio between the arc length and the width is at least 2:1, particularly at least 5:1, more particularly at least 10:1.

In particular, the curvature of the at least one hook extends outward from a distal face side of the leadless pacemaker in a lateral direction perpendicular to the longitudinal direction. This results in a tight anchorage of the pacemaker, particularly when a force is applied to the pacemaker along the longitudinal axis in the direction from the distal to the proximal end.

In certain embodiments, the communication unit is directly connected to the therapy unit, such that information is exchangeable between the communication unit and the therapy unit. In other words, the communication unit and the therapy unit are connected without an additional circuit or device therebetween. The direct connection between the communication unit and the therapy unit may be any suitable data connection known in the art.

In certain embodiments, the leadless pacemaker comprises an integration unit, wherein the communication unit is connected to the therapy unit via the integration unit, such that information is exchangeable between the communication unit and the therapy unit.

Information may comprise device specific information, like battery status and/or currently running stimulation program. Alternatively or in addition, information may comprise statistical information, e.g. about heart condition (for example occurrence of atrial fibrillation).

The integration unit may be any device suitable for mediating and/or controlling data transfer between the communication unit and the therapy unit. For example, the integration unit may be an electronic circuit.

In certain embodiments, the leadless pacemaker comprises a power supply which is connected to the communication unit and the therapy unit, such that electrical energy is provided to the communication unit and the therapy unit by means of the power supply.

In particular, the power supply is a battery and/or an energy harvesting unit. The term "energy harvesting unit" describes a device capable of accumulating electrical energy during operation of the leadless pacemaker. For instance, such an energy harvesting unit may comprise a mechano electric transducer such as a piezoelectric element configured to convert mechanical energy from movement during contraction of the heart to electrical energy.

In certain embodiments, the fixation element comprises or consists of an electrically conductive material, particularly a super-elastic material, more particularly a form-shape alloy such as, for example, Nitinol. Nitinol is a nickel titanium alloy commonly used in implants which is characterized by good shape memory and super-elastic characteristics.

A second aspect relates to a method for electrically stimulating cardiac tissue by means of a leadless pacemaker according to the first aspect, wherein the cardiac tissue is electrically stimulated by the fixation element, wherein the fixation element acts as an electrode (alone or in combination with other electrodes).

A third aspect relates to a method for sensing electrical signals of cardiac tissue by means of a leadless pacemaker according to the first aspect, wherein electrical signals of the cardiac tissue are sensed by the fixation element, wherein the fixation element acts as an electrode (alone or in combination with other electrodes).

In certain embodiments of the method for sensing electrical signals, a first electrical signal is sensed between a pacing electrode and the fixation element, and wherein a second electrical signal is sensed between the pacing electrode and a ring electrode of the leadless pacemaker, and wherein a far field signal of the cardiac tissue, particularly an atrial signal, is determined by an electronic circuit of the leadless pacemaker.

In particular, the pacing electrode is positioned at a distal end of the leadless pacemaker and the ring electrode is positioned at a proximal end of the leadless pacemaker.

A fourth aspect relates to a method for communicating between a leadless pacemaker according the first aspect and an external device, wherein signals generated by the communication unit are transmitted to the external device and/or signals received from the external device are transmitted to the communication unit by means of the fixation element, wherein the fixation element acts as a communication antenna.

All embodiments and features described above in the context of the first aspect also apply to and may be combined with the second to fourth aspect.

Wherever alternatives for single separable features are laid out herein as "embodiments", it is to be understood that such alternatives may be combined freely to form discrete embodiments of the invention disclosed herein.

The invention is further illustrated by the following examples and figures, from which further embodiments and advantages can be drawn. These examples are meant to illustrate the invention but not to limit its scope.
- Fig. 1: shows a first embodiment of the leadless pacemaker comprising hook-shaped fixation elements;
- Fig. 2: shows a second embodiment of the leadless pacemaker comprising hook-shaped fixation elements and an additional proximal ring electrode; and
- Fig. 3: shows a third embodiment of the leadless pacemaker comprising a fixation element shaped as a helical screw.

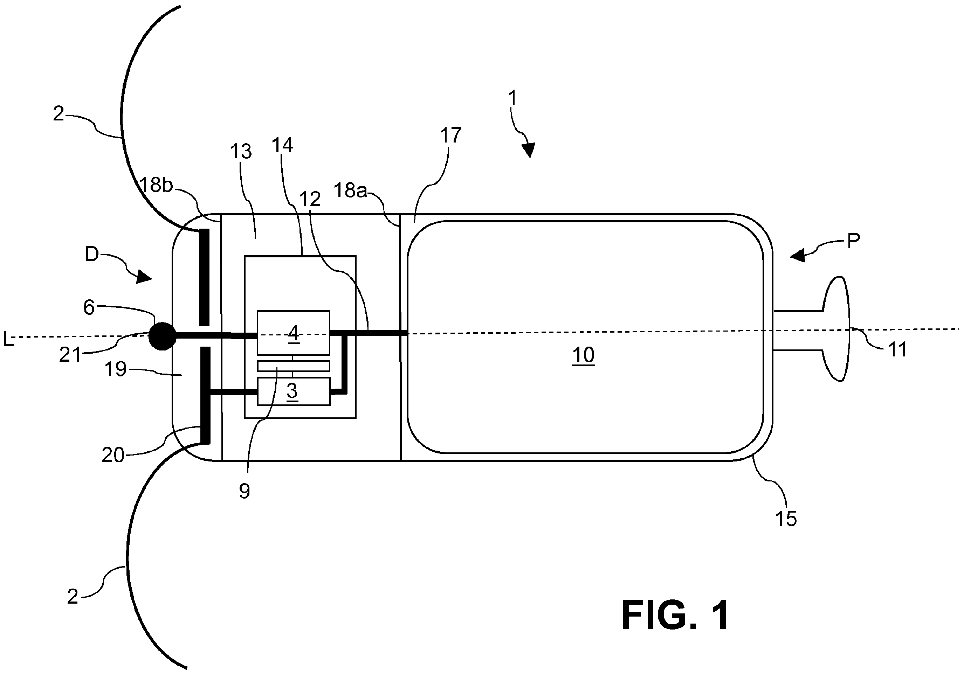

Fig. 1 schematically shows a sectional view of a leadless pacemaker 1 for implantation into human or animal cardiac tissue according to a first embodiment. The leadless pacemaker 1 extends along a longitudinal axis L from a distal end D, where the leadless pacemaker 1 is to be implanted into cardiac tissue, to a proximal end P. A housing 15, particularly from a metallic material, encases the internal components of the leadless pacemaker 1. At the proximal end P, an implant / explant port 11 is positioned.

The leadless pacemaker 1 may be installed for example in a ventricle or an atrium of the heart, particularly into an inner wall of the ventricle or atrium.

To fixate or anchor the leadless pacemaker 1 in the cardiac tissue at its desired position, the leadless pacemaker 1 comprises hook-shaped fixation elements 2 arranged at the distal end D. In the sectional view of Fig. 1, two fixation elements 2 are depicted, but the leadless pacemaker 1 may contain more than two fixation elements 2.

To provide electrical energy, the leadless pacemaker 1 comprises a power supply 10 positioned in a power supply compartment 17 within the housing 15 adjacent the proximal end P of the leadless pacemaker 1. In particular, the power supply 10 may be a battery. Instead of or in addition to a battery, the leadless pacemaker may contain an energy harvesting unit as a power source 10 or as part of the power source 10. Such an energy harvesting unit accumulates electrical energy, particularly by converting mechanical energy from motions occurring during beating of the heart to electrical energy.

The leadless pacemaker 1 further comprises an electronic compartment 13, adjacent to the power supply compartment 17, wherein the electronic compartment 13 is separated from the power supply compartment 17 by an inner wall 18a. In particular, the electronic compartment 13 is hermetically sealed from the adjacent compartments in particular to avoid fluid from entering the compartment and damaging the electronics.

Adjacent the electronic compartment 13, an electrode compartment 19 is positioned within the housing 15 at the distal end D of the leadless pacemaker 1. The electrode compartment 19 is separated from the electronic compartment 13 by inner wall 18b.

An electronic module 14, particularly for controlling pacing (electric pulse generation) and sensing of electrical signals of the heart, among other functions, is situated within the electronic compartment 13. The electronic module 14 is electrically connected to the power source 10 by means of electric connection 12, which protrudes through a feedthrough of the inner wall 18a separating the electronic compartment 13 from the power supply compartment 17.

The electronic module 14 comprises a communication unit 3 for communicating with an external device and a therapy unit 4 for generating electrical signals to electrically stimulate cardiac tissue in which the leadless pacemaker 1 is implanted. Both the communication unit 3 and the therapy unit 4 are electrically connected to power supply 10 by the electric connection 12 and thereby energized by the power supply 10.

In the example depicted in Fig. 1, the communication unit 3 and the therapy unit 4 are connected by means of an integration unit 9 allowing communication between the communication unit and the therapy unit 4.

The communication unit 3, which may be e.g. a transceiver, is electrically connected to the hook-shaped fixation elements 2 via electric connections 12 and an electrically conductive ring 20 to which the fixation elements 2 are mechanically connected. The ring 20 and a part of the hook-shaped fixation elements 2 are positioned within the electrode compartment 19 of the leadless pacemaker 1. The hook-shaped fixation elements 2 protrude through the housing 15 of the leadless pacemaker 1 to the exterior, where they are able to engage cardiac tissue when the leadless pacemaker 1 is implanted.

Due to their electrically conductive material and their connection with the communication unit 3, the hook-shaped fixation elements 2 serve as a communication antenna which is able to generate electromagnetic waves to transmit signals generated by the communication unit 3 to an external device, for example at frequencies within the MICS band (405MHz) or the Bluetooth frequency of 2.4 GHz. Furthermore, the fixation elements 2 are also configured to receive signals from an external device, such that they can be decoded and evaluated by the communication unit 3. This allows efficient wireless communication of the leadless pacemaker 1 with external devices.

The therapy unit 4 is electrically connected to a single pacing electrode 6, such that a positive or negative electrical potential can be applied to the pacing electrode 6 for electric stimulation of the cardiac tissue. The pacing electrode 6 shown in Fig. 1 is a tip electrode having a ball-shaped tip 21 with a convex surface to increase the electric field generated at the pacing electrode 6. The electrode compartment 19 harbors a part of the pacing electrode 6, and the pacing electrode 6 protrudes the housing 15 of the leadless pacemaker 1 via a feedthrough. The tip 21 of the pacing electrode 6 is positioned exterior to the leadless pacemaker and protrudes from the housing 15 of the device, such that the tip 21 is in direct contact with the cardiac tissue into which the leadless pacemaker 1 is implanted, and such that the pacing electrode 6 can electrically stimulate the cardiac tissue when an electric potential is applied to the pacing electrode 6.

In the embodiment shown in Fig. 1, the hook-shaped fixation elements 2, which are at ground potential, serve as a counter electrode for the pacing electrode 6 in addition to their function of fixating the leadless pacemaker 1 in the cardiac tissue and their function as a communication antenna to communicate wirelessly with external devices. Using the fixation elements 2 as a counter-electrode for the pacing electrode 6 advantageously improves the local resolution of the electrical stimulation of the heart.

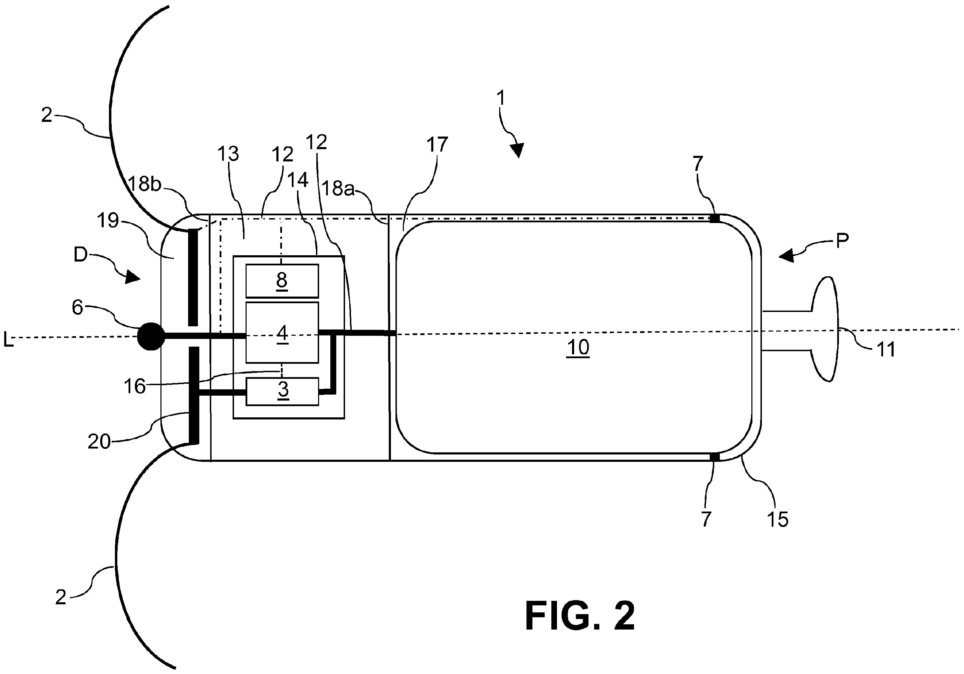

Fig. 2 illustrates an alternative embodiment of the leadless pacemaker 1 according to the invention. The pacemaker 1 is identical to the one depicted in Fig. 1 (see description above) except for the following additional features or alternative features.

In addition to the pacing electrode 6 and the hook-shaped fixation elements 2 serving as a counter electrode for the pacing electrode, the leadless pacemaker comprises a ring electrode 7 near the proximal end P of the leadless pacemaker 1. In the sectional view shown in Fig. 2, two segments of the ring electrode 7 are shown, but the ring electrode 7 extends circumferentially around the periphery of the housing 15. The ring electrode 7 serves as an additional counter electrode for the pacing electrode 6 and has a specialized function in signal sensing as described below.

The electronic module 14 of the leadless pacemaker 1 according to Fig. 2 comprises an additional electronic circuit 8, which is connected to the pacing electrode 6, the fixation elements 2 and the ring electrode 7. The electronic circuit 8 is configured to analyze a first electrical signal sensed between the pacing electrode 6 and the fixation element 2 and a second electrical signal sensed between the pacing electrode 6 and the ring electrode 7 and determine a far field signal, particularly generated in an atrium of the heart in case the leadless pacemaker is installed in the ventricle of the heart.

In contrast to setups known from the prior art, this allows to separate far field signals generated distant from the implantation site of the pacemaker 1 from near field signals resulting from cardiac tissue near the implantation site.

Furthermore, in contrast to the embodiment shown in Fig. 1, the communication unit 3 of the pacemaker 1 according to Fig. 2 is directly connected to the therapy unit 4 by a data connection 16.

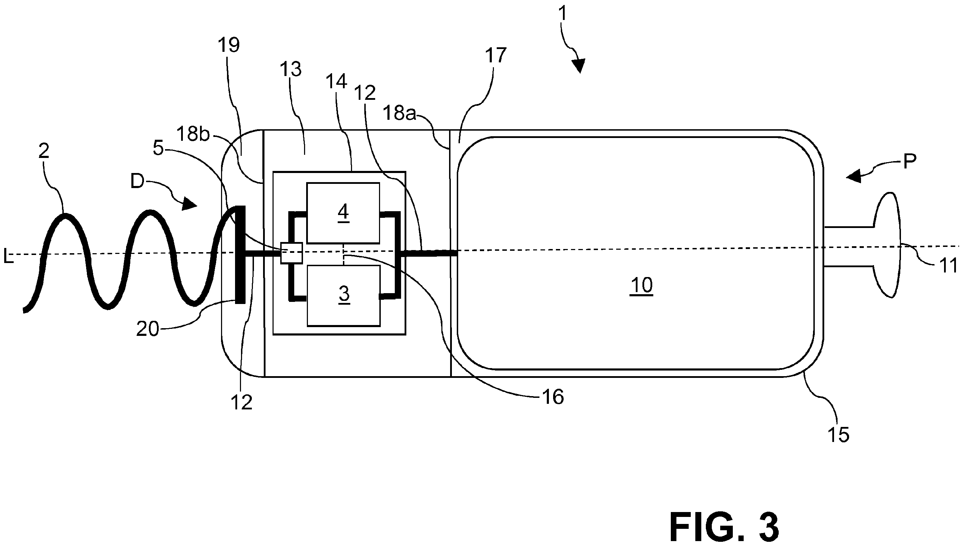

Fig. 3 shows a further embodiment of the leadless pacemaker 1 according to the present invention.

In contrast to the embodiments depicted in Fig. 1 and Fig. 2, the leadless pacemaker 1 according to Fig. 3 comprises a single fixation element 2 shaped as a helical screw extending along the longitudinal axis L from the distal end D of the leadless pacemaker 1. The pacemaker 1 can be anchored in the cardiac tissue at its desired location by screwing the fixation element 2 into the cardiac tissue.

Similar to the previously discussed embodiments, the screw-shaped fixation element 2 is connected to the communication unit 3, such that it may serve as a communication antenna to wirelessly transmit signals to and receive signals from an external device.

In addition, the screw-shaped fixation element 2 is electrically connected to the therapy unit 4, such that a positive or negative electric potential can be applied to the fixation element 2 relative to ground potential of the housing 15 or a separate ring electrode (not shown in Fig. 3, compare Fig. 2). In this manner, the fixation element 2 may be utilized as a pacing electrode, thereby eliminating the need for a designated pacing electrode 6 as shown in Fig. 1 and Fig. 2. This advantageously reduces weight and complexity of the leadless pacemaker 1.

To separate the signals from the communication unit 3 and the therapy unit 4, which are commonly characterized by separate frequencies, a signal splitter 5 is provided as part of the electronic module 14 of the leadless pacemaker 1 according to Fig. 3. The signal splitter 5 is electrically connected to both the communication unit 3 and the therapy unit 4, and electrically connected to the helical fixation element 2 via an electrical connection 12 and an electrically conductive ring 20 to which the fixation element 2 is mechanically connected. Alternatively, the signal splitter may also be positioned outside of the electronic module 14, even outside of the electronic compartment 13.

In the example shown in Fig. 3, the communication unit 3 is directly connected to the therapy unit 4 by data connection 16. Of course, it is also possible to connect the communication unit 3 to the therapy unit 4 via an integration unit 9, such as depicted in Fig. 1.

### List of reference numerals

- 1: Leadless pacemaker
- 2: Fixation element
- 3: Communication unit
- 4: Therapy unit
- 5: Signal splitter
- 6: Pacing electrode
- 7: Ring electrode
- 8: Electronic circuit
- 9: Integration unit
- 10: Power supply
- 11: Implant / explant port
- 12: Electric connection
- 13: Electronic compartment
- 14: Electronic module
- 15: Housing
- 16: Data connection
- 17: Power supply compartment
- 18a, 18b: Inner wall
- 19: Electrode compartment
- 20: Ring
- 21: Tip
- D: Distal end
- P: Proximal end
- L: Longitudinal axis

## Claims

1. A leadless pacemaker (1) comprising
- at least one fixation element (2) for fixating the leadless pacemaker to cardiac tissue,
- a communication unit (3) which is electrically connected to said fixation element (2), so that said fixation element (2) is configured to act as a communication antenna for transmitting signals generated by said communication unit (3) to an external device and/or receiving signals from an external device, and
- a therapy unit (4) for generating electrical signals to electrically stimulate cardiac tissue,
wherein said fixation element (2) is configured to act as an electrode for electrically stimulating cardiac tissue and/or sensing electrical signals of the cardiac tissue.

2. The leadless pacemaker (1) according to claim 1, wherein said fixation element (2) is electrically connected to said therapy unit (4), such that a voltage may be applied to said fixation element (2).

3. The leadless pacemaker (1) according to claim 2, wherein said leadless pacemaker (1) comprises a signal splitter (5) for separating signals of said communication unit (3) from signals of said therapy unit (4), wherein said fixation element (2) is electrically connected to said communication unit (3) and said therapy unit (4) via said signal splitter (5).

4. The leadless pacemaker (1) according to claim 1, wherein said leadless pacemaker (1) comprises a pacing electrode (6) which is electrically connected to said therapy unit (4), wherein said fixation element (2) is configured to act as a counter electrode, such that a voltage may be applied between said pacing electrode (6) and said fixation element (2).

5. The leadless pacemaker (1) according to claim 4, wherein said leadless pacemaker further comprises a ring electrode (7) and an electronic circuit (8), wherein said electronic circuit (8) is configured to determine a far field signal of the cardiac tissue from a first electrical signal sensed between said pacing electrode (6) and said fixation element (2) and a second electrical signal sensed between said pacing electrode (6) and said ring electrode (7), particularly wherein said pacing electrode (6) is positioned at a distal end (D) of the leadless pacemaker and said ring electrode (7) is positioned at a proximal end (P) of said leadless pacemaker (1).

6. The leadless pacemaker (1) according to any one of the preceding claims, wherein said fixation element (2) comprises a helical screw.

7. The leadless pacemaker (1) according to any one of the preceding claims, wherein said fixation element (2) comprises at least one hook for fixating the leadless pacemaker (1) to said cardiac tissue.

8. The leadless pacemaker (1) according to any one of the preceding claims, wherein said communication unit (3) is directly connected to said therapy unit (4), such that information is exchangeable between said communication unit (3) and said therapy unit (4).

9. The leadless pacemaker (1) according to any one of the preceding claims, wherein said leadless pacemaker (1) comprises an integration unit (9), wherein said communication unit (3) is connected to said therapy unit (4) via said integration unit (9), such that information is exchangeable between said communication unit (3) and said therapy unit (4).

10. The leadless pacemaker (1) according to any one of the preceding claims, wherein said leadless pacemaker (1) comprises a power supply (10) which is connected to said communication unit (3) and said therapy unit (4), such that electrical energy is provided to the communication unit (3) and the therapy unit (4) by means of the power supply (10).

11. The leadless pacemaker (1) according to any one of the preceding claims, wherein said fixation element (2) comprises or essentially consists of an electrically conductive material, particularly a superelastic material, more particular Nitinol.

12. A method for electrically stimulating cardiac tissue by means of a leadless pacemaker (1) according to any one of the preceding claims, wherein the cardiac tissue is electrically stimulated by said fixation element (2), and wherein said fixation element (2) acts as an electrode.

13. A method for sensing electrical signals of cardiac tissue by means of a leadless pacemaker (1) according to any one of claims 1 to 11, wherein electrical signals of the cardiac tissue are sensed by said fixation element (2), and wherein said fixation element (2) acts as an electrode.

14. The method according to claim 13, wherein a first electrical signal is sensed between a pacing electrode (6) and said fixation element (2), and wherein a second electrical signal is sensed between said pacing electrode (6) and a ring electrode (7) of said leadless pacemaker (1), and wherein a far field signal of the cardiac tissue, particularly an atrial signal, is determined by an electronic circuit (8) of said leadless pacemaker (1).

15. A method for communicating between a leadless pacemaker (1) according to any one of claims 1 to 11 and an external device, wherein signals generated by said communication unit (3) are transmitted to said external device and/or signals received from said external device are transmitted to said communication unit (3) by means of said fixation element (2), wherein said fixation element (2) acts as a communication antenna.
